# EUROPÄISCHE PATENTSCHRIFT

(11) **EP 0 637 376 B1**
(45) Veröffentlichungstag und Bekanntmachung des Hinweises auf die Patenterteilung: **05.03.1997**
(21) Anmeldenummer: 93907798.8
(22) Anmeldetag: 13.04.1993
(51) Int. Cl.: G01N 21/35

(54) **VERFAHREN UND ANORDNUNG ZUM MESSEN DER KONZENTRATION EINES NACHWEISGASES IN EINEM EIN STÖRGAS ENTHALTENDEN MESSGAS**
PROCESS AND DEVICE FOR MEASURING THE CONCENTRATION OF A DETECTOR GAS IN A MEASURING GAS CONTAINING AN INTERFERING GAS
PROCEDE ET DISPOSITIF POUR MESURER LA CONCENTRATION D'UN GAZ INDICATEUR DANS UN GAZ DE MESURE CONTENANT UN GAZ PERTUBATEUR

(30) Priorität: 21.04.1992 DE 4213051
(43) Veröffentlichungstag der Anmeldung: 08.02.1995
(73) Patentinhaber: SIEMENS AKTIENGESELLSCHAFT, 80333 München (DE)
(72) Erfinder: KIMMIG, Ludwig, D-7505 Ettlingen (DE)
(86) Internationale Anmeldenummer: DE9300333
(87) Internationale Veröffentlichungsnummer: WO9321515

(56) Entgegenhaltungen:
- EP-A- 0 343 143
- WO-A-89/03029
- DE-A- 4 021 864
- PATENT ABSTRACTS OF JAPAN vol. 9, no. 62 (P-342)(1785) 19 March 1985 & JP-A-59 196 461

## Beschreibung

Bei der nichtdispersiven Infrarot (NDIR)-Gasanalyse tritt das Problem der Querempfindlichkeit auf, wenn das Meßgas außer dem Nachweisgas, dessen Konzentration im Meßgas gemessen werden soll, ein Störgas enthält, dessen Absorptionsbanden denen des Nachweisgases weitgehend überlagert sind. Dies ist z. B. bei der Messung von Stickoxiden in Abgasen der Fall, die Wasserdampf als Störgas enthalten. Zur Beseitigung von Wasserdampf und auch anderen Störgasen ist es aus dem Buch "Messen, Steuern und Regeln in der chemischen Technik", 3. Auflage, Band II, Seite 611, Springer-Verlag 1980 bekannt, das Meßgas zu kühlen und anschließend über ein Trockenmittel, das von Zeit zu Zeit erneuert werden muß, zu leiten.

Auch in der internationalen Patentanmeldung WO 89/03029 ist angegeben, zum Messen von Stickoxiden im Abgas von Kraftfahrzeugmotoren das Meßgas zu kühlen, um den im Gas enthaltenen Wasserdampf zu entfernen und dadurch die Stickoxidmessung zu erleichtern. Die bekannten Maßnahmen zum Entfernen von Wasserdampf haben den Nachteil, daß ein aufwendiger Kühler notwendig ist, der einem Verschleiß unterworfen ist, und daß Betriebs- und Wartungskosten anfallen. JP-A-59 196 461 offenbart ein Gerät und Verfahren, in dem ein Störgas aus dem Meßgas entfernt und ein Nullgas mit Störgas beladen wird.

Der vorliegenden Erfindung liegt die Aufgabe zugrunde, ein Verfahren und eine Anordnung zum Messen der Konzentration eines Nachweisgases in einem ein Störgas enthaltenden Meßgas anzugeben, die im Vergleich zum Bekannten einfach und betriebssicher sind.

Erfindungsgemäß wird diese Aufgabe mit den in den kennzeichnenden Teilen der Ansprüche 1 und 4 angegebenen Maßnahmen gelöst.

Die Erfindung beruht auf dem Gedanken, dem Meßgas, dem Nullgas und dem Prüfgas so viel Störgas zuzusetzen, daß jeweils dieselbe Störgaskonzentration erreicht wird. Wenn die ursprüngliche Störgaskonzentration im Meßgas etwa gleich oder höher ist, kann es erforderlich sein, durch Kühlen die Konzentration herabzusetzen. Hierzu genügt im allgemeinen eine einfache Kühlung des Meßgases auf etwa die Umgebungstemperatur. Günstig ist es, wenn die ursprüngliche Störgaskonzentration in etwa erhalten bleibt oder nur wenig geändert wird, da durch Ändern der Störgaskonzentration auch die Konzentration des Nachweisgases beeinflußt wird, was im allgemeinen in einer Korrekturrechnung berücksichtigt werden muß.

Der mit dem Nullgas ermittelte Meßwert entspricht der Konzentration Null des Nachweisgases und bildet daher den Nullpunkt. Dieser Wert wird von den Meßwerten des Meßgases subtrahiert, und zwar entweder rechnerisch oder, bei Zweistrahl-NDIR-Analysatoren, physikalisch. Durch eine Messung des Prüfgases, welches das Nachweisgas in bekannter Konzentration enthält, wird die Steigung des Meßeffektes ermittelt.

Im Falle, daß die Konzentration von Stickoxiden in wasserdampfhaltigem Abgas mit einem Einstrahl-NDIR-Analysengerät gemessen wird, werden Meß-, Null- und Prüfgas nacheinander über einen einzigen Befeuchter geleitet. Dies hat den Vorteil, daß die Gase in gleicher Weise befeuchtet werden. Es muß aber von Zeit zu Zeit eine Meßpause eingelegt werden, in der Nullpunkt und Steigung neu bestimmt werden. Dies stört im allgemeinen nicht, da z. B. bei der Messung an Abgasen von Kraftfahrzeugmotoren die ohnedies notwendigen Meßpausen ausgenutzt werden können. Im Falle eines Zweistrahl-Analysengerätes kann das Nullgas als Vergleichsgas dienen, so daß der Nullpunkt nicht gesondert bestimmt zu werden braucht. Allerdings sind dann zwei Befeuchter erforderlich, die zweckmäßig baulich vereinigt sind, damit für die Befeuchtung weitgehend übereinstimmende Verhältnisse, z. B. gleiche Temperaturen, herrschen.

In einer vorteilhaften Ausführungsform enthält der Befeuchter eine Befeuchtungskammer, durch welche die Gase geleitet sind und die eine Befeuchtungswand aufweist, die aus einem Material besteht, das Wasser aufnimmt und an nicht mit Wasserdampf gesättigte Gase wieder abgibt. In einer besonders einfachen, für eine höhere Meßgenauigkeit jedoch weniger geeigneten Ausführungsform nimmt die Befeuchtungswand Wasserdampf aus dem Meßgas auf und gibt ihn an das Null- und das Prüfgas wieder ab. Dadurch wird der ursprünglich unterschiedliche Wasserdampfgehalt der Gase, z. B. das Abgas, Umgebungsluft und Prüfgas aus einer Flasche, ausgeglichen. Bevorzugt wird die Befeuchtungswand auf ihrer Rückseite mit Wasser in flüssiger oder dampfförmiger Form in Berührung gebracht, das sie aufnimmt und an das durch die Befeuchtungskammer strömende Gas abgibt.

Anhand der Zeichnung werden im folgenden die Erfindung sowie weitere Vorteile, Ausgestaltungen und Ergänzungen näher beschrieben und erläutert.
- Figur 1: zeigt schematisch eine Anordnung zum Messen der Konzentration von Stickoxiden in Abgasen.
- In Figur 2: ist im Querschnitt ein Gasbefeuchter dargestellt, der in der Anordnung nach Figur 1 alternativ eingesetzt werden kann.

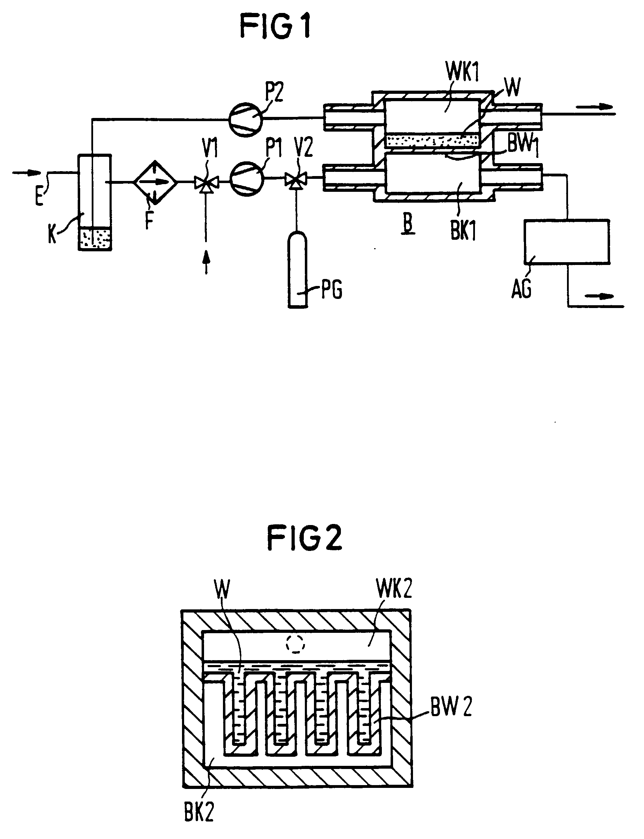

Der in Figur 1 dargestellten Anordnung wird über einen Eingang E Abgas aus einem Kraftfahrzeugmotor zugeführt. Das Abgas kühlt sich in der Leitung ab und gelangt in einen Kondensatabscheider K, an dessen Boden sich das Kondenswasser sammelt. Das von dem überschüssigen Wasserdampf befreite Abgas strömt durch ein Filter F und durch ein Ventil V1 zu einer Pumpe P1, die das Abgas durch ein Ventil V2 in eine Befeuchtungskammer BK1 eines Befeuchters B fördert. Von dort gelangt das Gas in einen NDIR-Analysator, mit dem der Stickoxidgehalt des Abgases gemessen wird.

Über der Befeuchtungskammer BK1 befindet sich im Befeuchter B eine Wasservorratskammer WK1, in die eine Pumpe P2 aus dem Kondensatabscheider K das Kondenswasser pumpt, das sich dort über einer Befeuchtungswand BW1 sammelt. Diese besteht aus einem Material, das Wasser aufnimmt und wieder abgibt, sofern es von nicht wasserdampfgesättigtem Gas umströmt wird. Im Ausführungsbeispiel ist als solches Material Polyamid eingesetzt, das zur Erhöhung der Festigkeit glasfaserverstärkt ist. Die Wand sollte sehr dünn, zweckmäßig eine Folie mit einer Stärke von weniger als 1 mm sein. Selbstverständlich können auch die übrigen Teile des Befeuchters B aus diesem Material bestehen. Auch kann der Befeuchter so aufgebaut sein, daß eine Befeuchtungskammer vollständig von Wasser umspült ist.

Das durch die Befeuchtungskammer BK1 strömende Meßgas nimmt von der Befeuchtungswand BW1 Wasserdampf bis zu einer bestimmten, im wesentlichen durch die Umgebungstemperatur gegebenen Konzentration auf. Selbstverständlich muß die Aufenthaltsdauer in der Befeuchtungskammer BK1 ausreichend lang sein. Es wird also gezielt Störgas dem Meßgas zugesetzt.

Zur Kompensation des durch das Störgas bedingten Meßeffektes im Analysengerät AG wird in einer Meßpause das Ventil V1 umgeschaltet und von der Pumpe P1 Umgebungsluft durch die Befeuchtungskammer BK1 zum Analysengerät AG gefördert. Diese Luft nimmt wie das Meßgas Feuchtigkeit auf.

Da sie im wesentlichen frei von Stickoxid ist, bestimmt allein der aufgenommene Wasserdampf den Meßeffekt. Subtrahiert man daher den dabei ermittelten Meßwert von den bei der Messung des Abgases gewonnenen Meßwerten, erhält man als Differenz einen Wert, welcher der Konzentration des Stickoxides entspricht.

Ein Gefäß PG enthält Prüfgas, das ist im vorliegenden Falle Stickoxid in bekannter Konzentration. Mit dessen Hilfe kann ein zweiter Punkt der Meßkennlinie der Meßanordnung ermittelt werden, indem das Ventil V2 umgeschaltet wird und das Prüfgas durch die Befeuchtungskammer BK1 dem Analysengerät AG zugeführt wird. Auch das Prüfgas nimmt dabei Feuchtigkeit auf, und zwar in derselben Konzentration wie das Meßgas und das Nullgas. Während der Meßwert des Nullgases den Nullpunkt der Meßkennlinie ergibt, wird aus den Meßwertprüfgasen die Steigung des Meßeffektes ermittelt.

Damit die die Befeuchtungskammer BK1 durchströmenden Gase ausreichend Feuchtigkeit aufnehmen, kann es erforderlich sein, die Oberfläche der Befeuchtungswand BW1 groß zu machen, indem sie z. B. aufgerauht wird. Eine andere Ausführungsform mit einer großen Oberfläche der Befeuchtungswand zeigt Figur 2, in der ein Querschnitt durch einen Befeuchter dargestellt ist. Dessen Befeuchtungswand BW2 ist mäanderförmig ausgebildet. Sie unterteilt den Befeuchter in eine Befeuchtungskammer BK2 und eine Wasservorratskammer WK2. Auf der Seite der Wasservorratskammer WK2 steht in den durch die Mäanderform gebildeten Schlitzen Wasser W, innerhalb der Befeuchtungskammer BK2 strömt durch die Mäanderschlitze das zu befeuchtende Gas. Anstelle der Mäanderform kann auch jede andere die Oberfläche vergrößernde Form gewählt werden.

## Patentansprüche

1. Verfahren zum Messen der Konzentration eines Nachweisgases in einem Störgas enthaltenden Meßgas, bei dem einem Analysengerät ein vom Nachweisgas freies Nullgas und das Meßgas zugeführt werden, wobei dem Nullgas Störgas zugesetzt wird, derart, daß die Störgaskonzentration im Nullgas gleich der in dem dem Analysator zugeführten Meßgas ist, **dadurch gekennzeichnet**, daß dem Meßgas, dem Nullgas und gegebenenfalls einem das Nachweisgas in bekannter Konzentration enthaltenden Prüfgas unter übereinstimmenden Verhältnissen Störgas zugesetzt wird.

2. Verfahren nach Anspruch 1, **dadurch gekennzeichnet**, daß aus dem Meßgas Störgas teilweise entfernt und dann bis zu einer bestimmten Konzentration zugesetzt wird.

3. Verfahren nach Anspruch 1 oder 2, **dadurch gekennzeichnet,** daß das Meßgas, das Nullgas und gegebenenfalls das Prüfgas nacheinander über dieselbe Einrichtung zum Zusetzen von Störgas geleitet werden.

4. Anordnung zum Messen der Konzentration eines Nachweisgases in einem Wasserdampf als Störgas enthaltenden Meßgas mit einem Zweistrahl-Analysengerät (AG), dem das Meßgas und ein vom Nachweisgas freies Nullgas zugeführt sind, und mit einem von Nullgas durchströmten Befeuchter, **dadurch gekennzeichnet**, daß in der Meßgaszuleitung ein zweiter Befeuchter (B) liegt, der mit dem vom Nullgas durchströmten Befeuchter baulich vereinigt ist.

5. Anordnung zum Messen der Konzentration eines Nachweisgases in einem Wasserdampf als Störgas enthaltenden Meßgas mit einem Analysengerät (AG), dem das Meßgas und ein vom Nachweisgas freies Nullgas zugeführt sind, und mit einem von Nullgas durchströmten Befeuchter, **dadurch gekennzeichnet,** daß eine Gasumschalteinrichtung (V1, V2) vorhanden ist, die das Meßgas, das Nullgas und gegebenenfalls ein Prüfgas mit bekannter Konzentration des Nachweisgases nacheinander durch einen einzigen Befeuchter (B) leitet.

## Claims

1. Method for measuring the concentration of a detection gas in a measuring gas containing interfering gas, where a zero gas free of the detection gas and the measuring gas are supplied to an analysis device, whereby interfering gas is added to the zero gas in such a way that the concentration of interfering gas in the zero gas is equal to that in the measuring gas supplied to the analyzer, characterized in that to the measuring gas, the zero gas and possibly a calibration gas containing the detection gas in known concentration interfering gas is added under corresponding conditions.

2. Method according to claim 1, characterized in that interfering gas is partially removed from the measuring gas and then added up to a certain concentration.

3. Method according to claim 1 or 2, characterized in that the measuring gas, the zero gas and possibly the calibration gas are conducted successively via the same device for the addition of interfering gas.

4. Arrangement for measuring the concentration of a detection gas in a measuring gas containing water vapour as interfering gas, having a two-beam analysis device (AG), which is supplied with the measuring gas and a zero gas free of the detection gas, and having a humidifier through which zero gas flows, characterized in that a second humidifier (B) lies in the measuring-gas supply line, which humidifier is structurally united with the humidifier through which the zero gas flows.

5. Arrangement for measuring the concentration of a detection gas in a measuring gas containing water vapour as interfering gas, having an analysis device (AG), which is supplied with the measuring gas and a zero gas free of the detection gas, and having a humidifier through which zero gas flows, characterized in that a gas changeover device (V1, V2) is present, which conducts the measuring gas, the zero gas and possibly a calibration gas with known concentration of the detection gas successively through a single humidifier (B).

## Revendications

1. Procédé pour mesurer la concentration d'un gaz indicateur dans un gaz de mesure contenant un gaz perturbateur, selon lequel on amène à un appareil d'analyse un gaz neutre exempt de gaz indicateur et le gaz de mesure, du gaz perturbateur étant ajouté au gaz neutre de telle sorte que la concentration en gaz perturbateur dans le gaz neutre est égale à celle dans le gaz de mesure amené à l'analyseur, caractérisé par le fait que l'on ajoute du gaz perturbateur, dans des conditions semblables, au gaz de mesure, au gaz neutre et éventuellement à un gaz de contrôle contenant le gaz indicateur dans une concentration connue.

2. Procédé selon la revendication 1, caractérisé par le fait que l'on élimine en partie du gaz perturbateur du gaz de mesure et on en ajoute ensuite jusqu'à une certaine concentration.

3. Procédé selon la revendication 1 ou 2, caractérisé par le fait que l'on amène successivement le gaz de mesure, le gaz neutre et éventuellement le gaz de contrôle, au même dispositif destiné à ajouter du gaz perturbateur.

4. Dispositif pour mesurer la concentration d'un gaz indicateur dans un gaz de mesure contenant de la vapeur d'eau comme gaz perturbateur, comportant un appareil d'analyse (AG) à double faisceau auquel sont amenés le gaz de mesure et un gaz neutre exempt de gaz indicateur, et comportant un humidificateur à travers lequel passe du gaz neutre, caractérisé par le fait qu'un second humidificateur (B), qui est réuni par sa structure à l'humidificateur à travers lequel passe le gaz neutre, se trouve dans la conduite d'amenée du gaz de mesure.

5. Dispositif pour mesurer la concentration d'un gaz indicateur dans un gaz de mesure contenant de la vapeur d'eau comme gaz perturbateur, comportant un appareil d'analyse (AG) auquel sont amenés le gaz de mesure et un gaz neutre exempt de gaz indicateur, et comportant un humidificateur à travers lequel passe du gaz neutre, caractérisé par le fait qu'il est prévu un dispositif de changement de gaz (V1, V2) qui conduit successivement à travers un seul humidificateur (B) le gaz de mesure, le gaz neutre et éventuellement un gaz de contrôle ayant une concentration connue de gaz indicateur.
